# EUROPEAN PATENT SPECIFICATION

(11) **EP 2 510 870 B1**
(45) Date of publication and mention of the grant of the patent: **13.07.2016**
(21) Application number: 12162600.6
(22) Date of filing: 30.03.2012
(51) Int. Cl.: A61B 5/00, G09F 3/00

(54) **A medical system and a method for operating a medical system**
Medizinisches System und Verfahren zur Bedienung des medizinischen Systems
Système médical et procédé de fonctionnement d'un système médical

(30) Priority: 15.04.2011 EP 11162641
(43) Date of publication of application: 17.10.2012
(73) Proprietor: F. Hoffmann-La Roche AG, 4070 Basel (CH); Roche Diagnostics GmbH, 68305 Mannheim (DE)
(72) Inventor: Thym, Detlef Dr., 68259 Mannheim (DE); Lorenz, Robert, 67547 Worms (DE)
(74) Representative: Bittner, Thomas L.

(56) References cited:
- WO-A1-2008/061313
- WO-A1-2009/053437
- WO-A2-2007/136902
- US-A1- 2006 265 246
- US-A1- 2010 327 057

## Description

The invention relates to a medical system and a method for operating a medical system.

### Background of the invention

Medical systems are used in order to perform a specific treatment and / or diagnostic step on patients. A large proportion of medical systems are contaminated in the course of such treatment or diagnostic step in that at least parts of the medical system come directly or indirectly into contact with the patient's body, in particular with bodily fluids.

This contamination can be caused by direct contact between parts of the medical system and the body of the patient. A contamination can however also arise in that a sample of bodily fluids taken from the body is placed in a test or analysis device at which point this test or analysis device can be contaminated with the bodily fluid. Such devices include, for example, blood sugar testing devices with which the blood sugar value is determined for blood samples of the patient. In connection with the extraction of blood samples, it is known that so-called lancing devices are used in order to pierce the surface of the skin of the patient for the purpose of taking the sample.

Document US 2009/0119024 A1 discloses a test system for measuring the concentration of an analyte in a biological fluid sample. A plurality of test members is provided in a container. A container is carrying a so called container RFID tag which includes data specific to said plurality of test members. In addition, a patient RFID tag is provided which includes data specific to a patient. A test meter configured for analyzing the biological fluid sample includes means for reading and storing data from the container RFID tag and from the patient RFID tag. The known system allows data pertaining to both the test members and the patient to be input quickly and easily to the meter without manual data entry, thereby reducing the likelihood of transcription errors and the time needed for data entry. If a different analyte test is to be carried out, a different container containing the appropriate test members will be used and the relevant container RFID data read by the meter.

Also, RFID test or RFID devices are used in other systems for analytical testing. Document US 2005/0009122 A1 discloses a system for analytical testing which comprises a test cartridge for use in conducting an analytical test including a test medium and an RFID device.

Document US 5,989,917 refers to a test meter of the type which receives a disposable test strip and a sample of bodily fluid from a patient and performs an electrochemical analysis of the amount of an analyte such as glucose in the sample. The test meter includes a receptacle for receiving a container in which disposable test strips are provided, and a magnetism for reading information about the disposable test strips that is affixed to the container. For example, calibration values can be applied to the container in the form of a machine readable barcode, a magnetic stripe, a memory chip or as a resonant wire loop.

In the document WO 2009/053437 A1 a medical system is proposed which comprises a central control unit equipped to display at least one diagnostic measured value by way of a display element. The medical system also comprises at least one invasive unit, said invasive unit having at least one invasive consumable. The invasive consumable is equipped to perform invasive surgery on a tissue of a patient. The invasive unit has at least one electronic identifier, which can be read out without contact, for storing at least one piece of information. The central control unit is equipped to electronically read out the at least one piece of information of the electronic indentifier.

Document US 2006/0265246 A1 discloses a medication safety system which includes a panel mounted to an IV pole above a multi-channel infusion pump also mounted to the IV pole. The panel includes multiple RFID readers for reading the RFID tags placed on each of the medication containers mounted to the panel. The pump includes a controller that communicates with the RFID readers at the panel to receive the information read by the RFID readers and automatically program the respective pump channel. A verification program verifies that the medication delivery information from the containers matches the patient identified for the pump and the programing parameters of the pump fall within the acceptable ranges. In this respect, upon obtaining the information from an information device of the container, a controller will compare it to a data base to determine if the delivery parameters of the information device fall within acceptable ranges in the data base. If so, the controller may then automatically program the respective pump with those parameters. If the parameters fall outside of a "soft" range or limit, the controller may provide a visual alert on a display and also an audible alert.

Document US 2010/0327057 A1 refers to maintaining sensor quality standards and / or preventing improper remanufacture of sensors, such as pulse oximetry sensors. A patient ID tag may be configured to attach to a patient and may include an optical machine-readable representation of data, such as a barcode. In one embodiment, the optical machine-readable representation of data is scanned by an optical reader. Encrypted data received in a memory may be compared to the data obtained by the optical scan, and, if the data does not match, the sensor may not function. This may prevent unauthorized remanufacture of the sensor because it would be difficult to perform bulk manufacturing operations.

There is need to further reduce the risk of infection during the use of such and other medical systems or even to completely rule it out. This applies in particular where the medical system is used for several patients. In such cases one must ensure that it is essential that a sufficient disinfection is undertaken prior to using the device for another patient.

### Summary of the invention

It is the object of the invention to provide improved technologies for a medical system and a method for operating a medical system which will reduce the risk of infection for the user.

This object is solved by a medical system according to the independent claim 1 and a method of operating a medical system according to the independent claim 13. Advantages on embodiments of the invention are the subject of dependent claims.

With the invention, a technology is provided by which a compulsory analysis is undertaken, prior to the use of a medical device with the help of a clearing device, for determining whether the medical device is prepared for a current use for the patient. For this analysis, use information is received from the medical device storage element by the clearing device. Also, identification information is received from the patient storage element by the clearing device. In a first step, the use information received from the medical device storage element is evaluated. If this shows, for example, that the medical device has previously been disinfected, the application on the patient can be cleared. Only when the analysis reveals that application on the patient is allowed a clearance signal will be output by the clearing device.

The clearance can occur without detailed analysis if the use information shows that the device is a new device which has not yet been used.

If in the first step of analyzing the use information it is found that application of the medical device to the patient is not allowed, e.g. because of prior use of the device, in a second step the identification information received from the patient storage element is analyzed. If in the second step it is found that application of the medical device to the patient is allowed, a clearance signal is output. If in an exemplary case a prior use of the medical device for a patient is indicated by the use information, the clearance can finally only occur if the respective patient in question is the one identified by the identification information.

The term "patient storage element" as used herein, especially, refers to a data storage element configured to be affixed to a patient's body or unequivocally assigned to a patient. Information stored on the data storage element can be read out by a contactless reading method, such as an optical and / or a non-optical reading or scanning procedure.

The term "medical device storage element" as used herein, especially, refers to a data storage element configured to be affixed or unequivocally assigned to a reusable medical device or instrument. Information stored on the data storage element can be read out by a contactless reading method, such as an optical and / or a non-optical reading or scanning procedure.

The term "a contaminating patient application" as used herein, especially, refers to an application of the device to a patient which includes a potential contact or the risk of a contact with a bodily fluid, especially blood, and thereby includes the risk of cross-contamination and / or the risk of a potential blood-borne infection, e.g. HIV or hepatitis B.

The clearance signal may be provided as a signal indicating to the user that use of the device is allowed. Such signal may be output by a signalling component of the clearance device itself. For example, an optical and / or an acoustic signalling component may be output. Also, as an alternative or in addition, such signalling to the user may be provided by the medical device. In addition or as an alternative, the clearing signal may comprise a signal component which is provided to the medical device for clearing operation of a component or a unit of the medical device or the medical device as whole, e.g. by clearing a blocking of the component and / or the medical device. In such case the clearing signal or its signal component may also be referred to as component / unit or device control or operation clearing signal. In one embodiment, clearing of a device component / unit and / or the device as whole may occur without outputting a signal informing the user. The user will learn that clearance has happened by noticing usability of the medical device.

The clearance signal can comprise acoustic and / or optical signals. Alternatively or in addition, a vibration signal could be produced as is known in the context of handheld devices such as mobile telephones. In this way, the user is signalled prior to using the medical device as to whether the current use of the medical device is allowed for patients or not. If, for example, the last use was for another patient, whereby the use information on the storage element of the medical device storage element identifies another patient, the clearance signal will not be emitted. Instead, a warning signal may be output.

In a preferred embodiment, the patient storage element is an electronic storage element which is loaded with electronic identification of the patient. The patient storage element being an electronic storage element or a different kind of storage element, preferably, may be worn on the body of the patient. In this way, information is available on the body of the patient if a use of the medical device is planned and prior to this the data on the patient storage element on the one side and on the medical device storage element on the other side must be analyzed with the help of the clearing device, in particular by the comparison of data. In another embodiment, the patient storage element is provided as a storage element which can be read out optically. For example, a storage element provided with a bar code and / or a 2D bar code and / or a hologram may be provided. In this case the clearing device will be provided with a reader module configured to read out the optical information.

Especially with respect to the medical device storage element, it is preferred to have the medical device storage element provided as a storage element accessible by non-optical read and / or write operations, such as RFID technology or electronic data memory devices. The medical device storage element, optionally also the patient storage element, may be implemented as a storage element configured to contain electronic information or data.

If both the patient storage element and the medical device storage element are configured to contain electronic information or data, a medical system is provided, comprising a patient storage element, the patient storage element being assigned to a patient by comprising electronic identification data identifying the patient, a medical device, the medical device being configured for a contaminating patient application, a medical device storage element, the medical device storage element being provided on the medical device and comprising electronic use information, and a clearing device, the clearing device being configured to receive the identification information from the patient storage element and the use information from the medical device storage element, analyze the use information, and, if in the step of analyzing the use information it is found that application of the medical device to the patient is allowed, output a clearance signal, and, if in the step of analyzing the use information it is found that application of the medical device to the patient is not allowed, analyze the identification information, and, if in the step of analyzing the identification information it is found that application of the medical device to the patient is allowed, output the clearance signal.

If both the patient storage element and the medical device storage element are configured to contain electronic information or data, a method may be implemented as follows: storing electronic identification data on a patient storage element, the electronic identification data identifying a patient, providing a medical device storage element on a medical device, the medical device being configured for a contaminating patient application, storing electronic use information on the medical device storage element, providing a clearing device, and in the clearing device, the method further comprising the following steps of receiving the identification information from the patient storage element and the use information from the medical device storage element, analyzing the use information, and, if in the step of analyzing the use information it is found that application of the medical device to the patient is allowed, output a clearance signal, and, if in the step of analyzing the use information it is found that application of the medical device to the patient is not allowed, analyze the identification information, and, if in the step of analyzing the identification information it is found that application of the medical device to the patient is allowed, output the clearance signal.

If the clearing device signals a clearance, the medical device may be used for the patient. In the course of such use or in the course of a first use, in a possible embodiment, information about the patient is automatically stored on the medical device storage element which identifies the patient for which the use then currently occurs. This can, for example, be provided in that electronic identification data stored on the patient storage element is transferred in full or in part to the medical device storage element. Alternatively or additionally, it can be provided that, with the help of the clearing device, information on the prior usages of the medical device for the patient is also stored on the medical device storage element. Thereby, the electronically stored information in the medical device storage element also comprises further electronic identification data which is then analysed by the clearing device prior to the next use of the medical device.

A medical device in the sense used here is a device configured for a contaminating medical patient application, be it on the basis of direct contact with the body of the patient or indirect contact, for example through a bodily fluid sample of the patient being analysed or determined with the help of the medical device. The latter is, for example, the case for a blood sugar testing device.

In still another preferred embodiment, the use information comprises at least one kind of information selected from the following group: further identification information, disinfection information, and non-use information. The further identification information may be provided as further electronic identification data. In a preferred embodiment, there is disinfection information stored on the medical device storage element. The disinfection information may be provided on the medical device storage element in addition or alternatively to further identification information. In case of use of the clearing device, the clearing signal may be output immediately if the disinfection information is detected on the medical device storage element. This is because the medical device is ready for use in view of the disinfection procedure done before. In such a case there needs to be no further analysis of the identification information, since the identity of the patient does not matter because of the disinfection. Therefore, analysis of the identification information is optional in such situations. Also, there could be information on the medical device storage element indicating that the medical device has not been in use for contaminating patient application at all before; such information may be referred to as non-use information. The non-use information, for example, may indicate that there is a new medical device which has not been applied to any patient before. In such a case, also, the clearing signal may be outputted without further data analysis, since there is no risk of infection due to prior use with some other patient. Again, analysis of the electronic identification data is optional in such situations.

In a preferred embodiment, the clearing device is at least partially implemented as an integrated device together with one of the patient storage element and the medical device storage element. In this embodiment, the clearing device is implemented with its components, fully or in part together with the patient storage element and / or the medical device storage element. The clearing device may also be referred to as a clearing module or clearing component. In this way, it is made possible for the clearing device to be integrated into the medical device so that a type of combination device is created. It can be provided in one embodiment that the clearing device becomes an integral part of a blood sugar testing device with which a blood sugar or glucose value is determined for a blood sample. The blood sugar testing device can in turn, in this or another design, be part of a system which also comprises a lancing device and / or a so-called insulin pen, thus an apparatus for administering insulin. The clearing device can also be formed in a device or system unit with the patient storage element. In this way, such a unit can be worn on the body of the patient in that the unit is attached to a wrist or ankle band which is removably attached to the body of the patient, for example using a suitable hook and loop fastening.

In still a further embodiment, at least one of the patient storage element and the medical device storage element is a RFID storage element. Such storage elements form so-called transponders which are known as such in various embodiments. The data transfer occurs in the case of RFID technology, via radio waves, that is to say wireless data communications.

In another preferred embodiment, at least one of the patient storage element and the medical device storage element is a passive storage element. The patient storage element can be a read only memory (ROM). The medical device storage element is preferably a read and write memory. In this way, it is made possible for the medical device storage element to be written over with electronic data, for instance after being disinfected or applied to a patient.

According to a preferred embodiment, at least one of the patient storage element and the medical device storage element is an active storage element. As used here, an active storage element is connected to its own energy source. Therefore, the active storage element is able to actively transmit stored electronic data. For example, the patient storage element may actively send out the electronic identification data identifying the patient to the clearing device. In addition or alternatively, the medical device storage element may be implemented as an active storage element being able to actively transmit the electronic use information to the clearing device. Such active data transmission may be started in at least one of the storage elements in response to detection of the clearing device in the vicinity of the location of one of the storage elements. Also, an active transmission of the electronic data identifying the patient to the medical device storage element may be started in response to detection of the medical device storage element in the vicinity of the location of the patient storage element. Thereby, electronic information about (potential) application of the medical device to the patient identified by the electronic identification data on the patient storage element is automatically transferred to the medical device storage element.

In still a further embodiment, the patient storage element is provided on a patient attachment element. The patient attachment element may be provided as a disposable article. The patient storage element may be detachably or non-detachably connected to the patient attachment element and can be constructed in a way that it can be removed without any destruction or alternatively only through destruction of the function of the disposable storage element. For example, the patient attachment element can be a wrist or ankle band which is detachably connected to the respective place on the body. An alternative embodiment may provide that the patient storage element is attached to the body using an adhesive, for example on a fingernail or toenail, on the hair or another body part. An adhesive could be used on a fingernail or toenail, for example, a superglue. The wrist or ankle band can have any form. In this way, for example, plastic materials may be used whose ends are detachably or non-detachably connected to one another. A non-detachable connection can be realized, for example, through heat sealing or thermal riveting or irreversible snap joint. It can also be provided in one embodiment that the patient storage element, for example a RFID chip, is implanted under the skin.

In one embodiment, the medical device storage element is a disposable article detachably applied to the medical device. The detachable connection of the storage element to the medical device, for example, makes it possible that the medical device storage element can be replaced by a new disposable storage element after a disinfection procedure. A detachable connection can be achieved through the use of a suitable adhesive. In one preferred embodiment the medical device storage element is a label with an integrated RFID and an adhesive for attachment. Such a label, for example, can be constructed in a way that it can be removed without any destruction or alternatively only through destruction of the function of the disposable storage element. A mechanical connection, for example a reversible or irreversible snap joint, can also be used for this. Alternatively, it can be provided that the medical device storage element is non-detachably connected to the medical device. Such a connection can, for example, be achieved through a plastic band or a shrink film so that the medical device storage element is only removable through the destruction of the means of attachment and optionally through the destruction of the function of the disposable storage element. Connection with so-called super glue can also be provided. In one embodiment it can be provided that the medical device storage element is integrated into the housing of the medical device.

As an alternative to the replacement of the medical device storage element after disinfection, it can be provided that the medical device storage element is electronically reset so that data related to the use for the patient stored thereon is deleted. The medical device is thus ready for use on the same patient for which the medical device was previously used or another patient. If such reset storage is detected with the help of the clearing device, a clearing signal is output.

In another preferred embodiment, at least one of the patient storage element and the medical device storage element is functionally connected to a motion sensor. The functionally or operationally coupling of the respective storage element to a motion sensor enables an operation mode in which, for example, the sending and / or reception of electronic data by the storage element is automatically initiated if it is moved. In this way, for instance, it can be provided, in connection with the medical device storage element that this element automatically sends the electronic use information if motion is detected by a respective motion sensor. The data transmitted can then be received by the clearing device. In a similar way, this can be provided for the patient storage element. Alternatively or in addition, the clearing device can be provided with a motion sensor so that a type of use for the clearing device is possible for which the clearing device is activated through detected motion in order to receive data from storage elements in the vicinity whereby a clearing process may be automatically initiated.

According to a preferred embodiment, the clearing device comprises a detection element and is configured to start, if at least one of the patient storage element and the medical device storage element is detected by the detection element as being located in the vicinity of the medical device, an operation mode in which the identification information and the use information are collected automatically by the clearing device from the patient storage element and the medical device storage element, respectively. In this mode of operation the electronic identification information and the electronic use information are collected or gathered by the medical device without waiting for a user input usually initiating such data collection. For example, the automatic data collection may be started in response to a signal received from a motion sensor.

In still a further embodiment, the clearing device is functionally connected to an operation module of the medical device, the clearing device being further configured to provide, if in the step of analyzing it is found that application of the medical device to the patient is allowed, the clearance signal with an operation clearing signal which is provided to the operation module for clearing use of an operation function of the medical device. In this embodiment the clearing signal is provided with an operation clearing signal which clears operation of a function and / or a functional element of the medical device. In case there is no allowed application to the patient, e.g. no patient identity, a locking signal may be provided which will lock the respective operation function against use. For example, in a medical device being provided as a testing or analyzing device for a bodily fluid such operation function may be any required preparation or functional step prior to application of bodily fluid sample. Such a required preparation or functional step could be, for example, providing a required testing element (such as a test strip), clearing an unused testing element for sample application or a display message. In case the locking signal is dispatched such operation function is blocked. In another embodiment, the medical device may be a puncturing device for puncturing the skin of the patient. If the locking signal is dispatched, the puncturing function is blocked. In another embodiment, the medical device may be provided as a so-called insulin pen which is a device for injecting insulin. If a criterion for allowing use is lacking, e.g. identity of the patient identifying information, clearance of the cannula is blocked by the locking signal. The aforementioned embodiments may preferably be implemented by combining the medical device and the clearing device in an integrated device.

According to a further embodiment, the medical device is a device or a combination of devices selected from the following group: a lancing device for puncturing a patient skin, a sample taking device for taking a bodily fluid sample, a testing device for determining a bodily fluid, and a medical injection device for administering fluids parenterally (such as a so-called insulin pen). The sample taking device, for example, may be configured for taking a blood sample from the patient. In a preferred embodiment, the testing device is configured for determining a blood sugar value for a blood sample of the patient.

With respect to the method for operating a medical device, the preferred embodiments described above may apply accordingly.

Dependent on the respectively chosen construction of the storage element, different forms of the method can be provided. The embodiment of the patient storage element and / or the medical device storage element as an active storage element thus provides for the active transmission of the electronic data by the storage element, in particular to the clearing device. Through the possible use of one or more motion sensors, method steps can be automatically initiated when motion is detected.

In one embodiment of the method it can be provided that an analysis may be performed, with the help of the clearing device, as to the usability for one patient of several components of a medical device and / or several medical devices. In this way, it can be provided, in connection with a blood sugar testing device, that the analysis may be performed for a testing device and also a lancing device whereby both the testing device and the lancing device are both provided with an allocated electronic storage element. In addition to or as an alternative to the analysis for the lancing device, an analysis for a so-called insulin pen can be provided. In this context, one embodiment of the invention provides that the clearing signal is only output if usability is determined for several components of a medical device or several medical devices. In another case it can be provided that components of the medical device or devices can be blocked and / or a warning signal emitted. Such blocking may be implemented by actively blocking in case there is no clearance or by not clearing a blocking status which may be referred to as passive blocking. The implementations described are, naturally, also possible in the case of a procedure with only one medical device.

### Description of preferred embodiments of the invention

In the following the invention will be described in further detail, by way of example, with reference to different embodiments. In the figures show:
- Fig. 1: a schematic representation of a medical system comprising a medical device,
- Fig. 2: a schematic representation of another medical system comprising a medical device which is provided with a motion sensor, and
- Fig. 3: a schematic representation of another medical system, the medical device being provided with a lancing device.

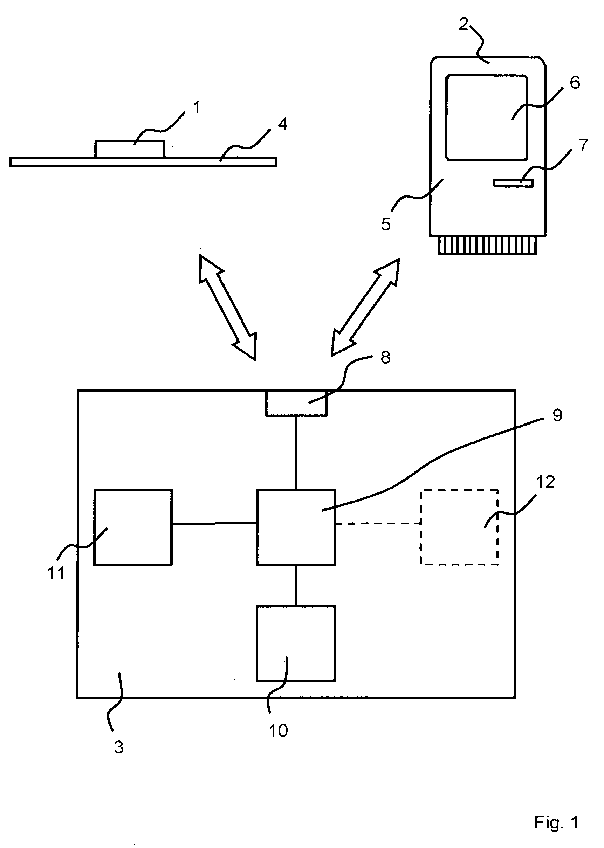

Fig. 1 shows a schematic representation of a medical system comprising a patient storage element 1, a medical device 2, and a clearing device 3.

The patient storage element 1 is arranged on a fastening element 4, for example a tie strap, with which a patient can attach the patient storage element 1 to his body. The patient storage element 1 has identification information, preferably electronic identification data, loaded onto it which distinctly identify the patient or user who is wearing the patient storage element 1 on his body with the help of the fastening element 4. For this, any identification data can be stored on the patent storage element which is suitable for identifying the patient distinctly from other patients. In a preferred embodiment, the patient storage element 1 is a so-called RFID transponder, that is a storage element with which data transfer by means of RFID technology may be undertaken.

In the embodiment shown, the medical device 2 is a blood sugar testing device with which the blood sugar value is determined, for previously extracted blood samples of a patient. In the embodiment example shown, a medical device storage element 7 is arranged on the housing 5 of the medical device 2 below a display 6. The medical device storage element 7 stores electronic data and is also provided as an RFID transponder in the example shown.

Both the patient storage element 1 and the medical device storage element 7 can be read out with the help of the read-write-unit 8 of the clearing device 3. The electronic data received by the clearing device 3 in this way are processed with a control unit 9. Depending on the processing steps involved, the control unit 9 effects the output of signals via an output element 10 of the clearing device 3. Acoustic and / or optical signals can be output. It can also be provided that the clearing device 3 can output vibration signals.

According to Fig. 1, the clearing device 3 has a storage unit 11 which can be used to store electronic data. Further components can be provided in the clearing device 3, which is shown by the component 12 outlined with dotted lines.

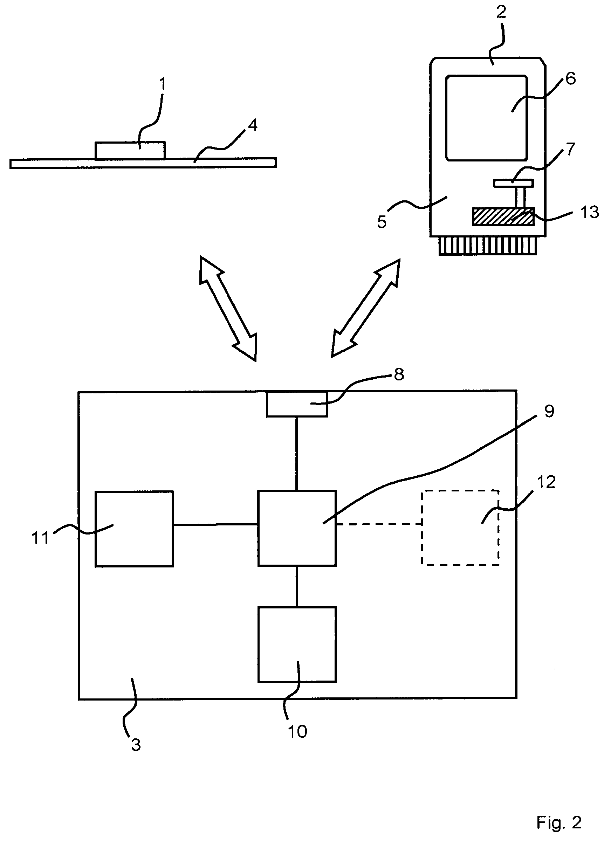

Fig. 2 shows a schematic representation of another medical system in which the medical device 2 additionally comprises a motion sensor 13 which is connected to the medical device storage element 7. The motion sensor 13 is functionally coupled to the medical device storage element 7. If the medical device storage element 7 is implemented as an active storage element there is an operation mode in which electronic data stored in the medical device storage element 7 are automatically transmitted when a motion of the medical device 2 is detected by the motion sensor 13. Thereby, electronic data stored in the medical storage element 7 may be transferred from the medical device 2 to the clearing device 3 without receiving a user input either in the medical device 2 or the clearing device 3.

With the help of the medical systems in Fig. 1 and 2, different methods of operation with different types of use can be provided. Such types of use are described by way of example in greater detail in the following.

In a method for the operation of the medical system, the medical device 2 embodied as a blood sugar testing device is used to determine a blood sample of a patient who is wearing the patient storage element 1 and who is identifiable with the help of electronic identification data stored on the patient storage element 1. In the course of the blood sugar analysis, the physical proximity of the patient storage element 1 and the medical device 2 with attached medical device storage element 7 causes the partial or complete transfer of the electronic data identifying the patient from the patient storage element 1 to the medical device storage element 7. In this way, electronic information is stored on the medical device storage element 7 that the medical device 2 was used in a contaminating manner for the patient identifiable through the electronic identification data on the patient storage element 1.

If the medical device 2 is later to be used for a new blood sugar analysis, the electronic identification data on the patient storage element 1 and the electronic data on the medical device storage element 7 are read and compared by the clearing device 3. As according to the above described procedure, the electronic data in the patient storage element 1 on the one side and the medical device storage element 7 on the other identify the same patient, the clearing device 3 subsequently outputs a clearance signal. For example, a green light could illuminate. In addition or as an alternative, an acoustic signal can be output or text via a display. In this way the user of the medical system has information that a renewed use of the medical device 2 is possible in connection with the patient who has been identified using the electronic identification data.

If it is determined in the course of such an analysis of the electronic identification data in the clearing device 3 that the data read out does not identify the same patient, a warning signal is output. For example, a red light could flash. In addition or as an alternative, an acoustic warning signal, a vibration warning signal can be output or warning text via a display. Such a case could occur, for example, if the medical device 2 previously used in the manner described above for analyzing blood sugar, is now to be used for another patient who is identified through the electronic identification data attributed to him. The output of a warning signal ensures that the contaminated medical device 2 is not used for the other patient.

This does not occur if the medical device 2 which has previously been used for one patient has been disinfected or sterilised before the intended use for another patient. In this case, the data stored on the medical device storage element 7 during the previous use pertaining to the one patient is erased so that the medical device storage element 7 no longer contains this data. Then, when the clearing device 3 reads the medical device storage element on the one side and the patient storage element on the other, clearance can be immediately given as the lack of personal identification data on the medical device storage element 7 shows that the medical device 2 may be used for the other patient.

In connection with the sterilisation or disinfection process, it can also be provided that the medical device storage element 7 is replaced by a new storage element, for example, by removing the previously used storage element and affixing a new storage element. In this way, the new storage element would also be free of personal identification data which would also allow the output of a clearance signal when checked using the clearing device 3.

It can optionally be provided, in connection with a sterilization or disinfection process, that electronic data about such a process is saved on the medical device storage element 7. This information can then be read by the clearing device 3 in the analysis process. Ultimately it shows that the medical device 2 is available for use for another patient.

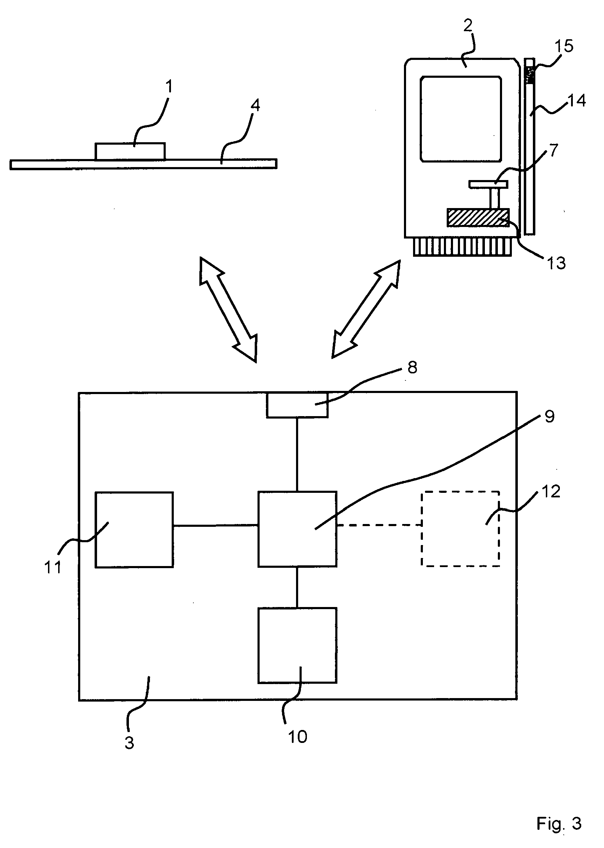

Fig. 3 shows a schematic representation of another medical system, wherein the medical device 2 is provided with a lancing device 14 which may be a separate detachable unit and may be referred to as another medical device. The lancing device 14 is configured for puncturing the skin of a patient. The lancing device 14 is provided with a further medical device storage element 15 separated from the medical device storage element 7.

In the scope of the method described above, it can be provided in the further medical system in Fig. 3 that when checking the patient identity the further medical device storage element 15 is also read on which the patient identifying data has been stored in a similar way to on the medical device storage element 7. It can be provided that the clearance signal is only output by the clearing device 3 if clearance is detected for both the medical device 2 and the further medical device 14. Separate signal outputs can also be provided for the medical device 2 and the further medical device 14 so that the situation can arise that a clearance signal is output for the medical device 2 but a warning or blocking signal is output for the further medical device 14 as the comparison of data on the further medical device storage element 15 with that on the patient storage element 1 did not reveal patient identity. This then means that the medical device 2 implemented as a blood sugar testing device is allowed to be used for a blood sugar analysis for the identified patient whereby further medical device 14, implemented as a lancing device may not be used.

## Claims

1. A medical system, comprising:
- a patient storage element (1), the patient storage element (1) being assigned to a patient by comprising identification information identifying the patient,
- a medical device (2), the medical device (2) being configured for a contaminating patient application,
- a medical device storage element (7), the medical device storage element (7) being provided on the medical device (2) and comprising use information, and
- a clearing device (3), the clearing device (3) being configured to
- receive the identification information from the patient storage element (1) and the use information from the medical device storage element (7), and
- analyze the use information,
**characterized in that** the clearing device (3) is further configured to
- if in the step of analyzing the use information it is found that application of the medical device (2) to the patient is allowed, output a clearance signal, and
- if in the step of analyzing the use information it is found that application of the medical device (2) to the patient is not allowed, analyze the identification information, and, if in the step of analyzing the identification information it is found that application of the medical device (2) to the patient is allowed, output a clearance signal.

2. System according to claim 1, wherein the use information comprises at least one kind of information selected from the following group: further identification information, disinfection information, and non-use information.

3. System according to claim 1 or 2, wherein the clearing device (3) is at least partially implemented as an integrated device together with one of the patient storage element (1) and the medical device storage element (7).

4. System according to one of the preceding claims, wherein at least one of the patient storage element (1) and the medical device storage element (7) is a RFID storage element.

5. System according to one of the preceding claims, wherein at least one of the patient storage element (1) and the medical device storage element (7) is a passive storage element.

6. System according to one of the preceding claims, wherein at least one of the patient storage element (1) and the medical device storage element (7) is an active storage element.

7. System according to one of the preceding claims, wherein the patient storage element (1) is provided on a patient attachment element (4).

8. System according to one of the preceding claims, wherein the medical device storage element (7) is a disposable article detachably applied to the medical device (2).

9. System according to one of the preceding claims, wherein at least one of the patient storage element (1) and the medical device storage element (7) is functionally connected to a motion sensor.

10. System according to one of the preceding claims, wherein the clearing device (3) comprises a detection element and is configured to start, if at least one of the patient storage element (1) and the medical device storage element (7) is detected by the detection element as being located in the vicinity of the medical device (2), an operation mode in which the identification information and the use information are collected automatically by the clearing device (3) from the patient storage element (1) and the medical device storage element (7), respectively.

11. System according to one of the preceding claims, wherein the clearing device (3) is functionally connected to an operation module of the medical device (2), the clearing device (3) being further configured to provide, if in the step of analyzing it is found that application of the medical device (2) to the patient is allowed, the clearance signal with an operation clearing signal which is provided to the operation module for clearing use of an operation function of the medical device (2).

12. System according to one of the preceding claims, wherein the medical device (2) is a device or a combination of devices selected from the following group: a lancing device for puncturing a patient skin, a sample taking device for taking a bodily fluid sample, a testing device for testing a bodily fluid, and a medical injection device for administering a fluid parenterally.

13. A method for operating a medical system (2), the method comprising steps of:
- providing a patient storage element (1), the patient storage element (1) comprising identification information identifying a patient,
- providing a medical device storage element (7) on a medical device (2), the medical device (2) being configured for a contaminating patient application,
- providing use information on the medical device storage element (7),
- providing a clearing device (3), and
- in the clearing device (3), the method further comprising the following steps of
- receiving the identification information from the patient storage element (1) and the use information from the medical device storage element (7), and
- analyzing the use information,
**characterized by**, in the clearing device (3), the method further comprising the following steps of
- if in the step of analyzing the use information it is found that application of the medical device (2) to the patient is allowed, outputting a clearance signal, and
- if in the step of analyzing the use information it is found that application of the medical device (2) to the patient is not allowed, analyzing the identification information, and, if in the step of analyzing the identification information it is found that application of the medical device (2) to the patient is allowed, outputting a clearance signal.

## Patentansprüche

1. Medizinisches System, aufweisend:
- ein Patienten-Speicherelement (1), wobei das Patienten-Speicherelement (1) einem Patienten durch Umfassen von Identifizierungsinformationen, die den Patienten identifizieren, zugeordnet ist,
- eine medizinische Vorrichtung (2), wobei die medizinische Vorrichtung (2) für eine kontaminierende Patientenanwendung konfiguriert ist,
- ein medizinisches Vorrichtungs-Speicherelement (7), wobei das medizinische Vorrichtungs-Speicherelement (7) auf der medizinischen Vorrichtung vorgesehen ist, (2) und Verwendungsinformationen umfasst, und
- eine Freigabevorrichtung (3), wobei die Freigabevorrichtung (3) konfiguriert ist,
- die Identifizierungsinformationen von dem Patienten-Speicherelement (1) und die Verwendungsinformationen von dem medizinischen Vorrichtungs-Speicherelement (7) zu empfangen und
- die Verwendungsinformationen zu analysieren,
**dadurch gekennzeichnet, dass** die Freigabevorrichtung (3) ferner konfiguriert ist
- sofern im Schritt des Analysierens der Verwendungsinformationen gefunden wird, dass die Anwendung der medizinischen Vorrichtung (2) für den Patienten erlaubt ist, ein Freigabesignal auszugeben, und
- sofern im Schritt des Analysierens der Verwendungsinformationen gefunden wird, dass die Anwendung der medizinischen Vorrichtung (2) für den Patienten nicht erlaubt ist, die Identifizierungsinformationen zu analysieren, und, sofern in dem Schritt des Analysierens der Identifizierungsinformationen gefunden wird, dass die Anwendung der medizinischen Vorrichtung (2) für den Patienten erlaubt ist, ein Freigabesignal auszugeben.

2. System gemäß Anspruch 1, wobei die Verwendungsinformationen mindestens eine Art von Informationen umfassen, die aus der folgenden Gruppe ausgewählt sind: weitere Identifizierungsinformationen, Desinfektionsinformationen und Nichtverwendungsinformationen.

3. System gemäß Anspruch 1 oder 2, wobei die Freigabevorrichtung (3) mindestens teilweise als eine integrierte Vorrichtung mit dem Patienten-Speicherelement (1) und / oder dem medizinischen Vorrichtungs-Speicherelement (7) implementiert ist.

4. System gemäß einem der vorhergehenden Ansprüche, wobei das Patienten-Speicherelement (1) und / oder das medizinische Vorrichtungs-Speicherelement (7) ein RFID-Speicherelement ist.

5. System gemäß einem der vorhergehenden Ansprüche, wobei das Patienten-Speicherelement (1) und / oder das medizinische Vorrichtungs-Speicherelement (7) ein passives Speicherelement ist.

6. System gemäß einem der vorhergehenden Ansprüche, wobei das Patienten-Speicherelement (1) und / oder das medizinische Vorrichtungs-Speicherelement (7) ein aktives Speicherelement ist.

7. System nach einem der vorhergehenden Ansprüche, wobei das Patienten-Speicherelement (1) an einem Patienten-Befestigungselement (4) vorgesehen ist.

8. System gemäß einem der vorhergehenden Ansprüche, wobei das medizinische Vorrichtungs-Speicherelement (7) ein Einwegartikel ist, der lösbar an der medizinischen Vorrichtung (2) angebracht ist.

9. System gemäß einem der vorhergehenden Ansprüche, wobei das Patienten-Speicherelement (1) und / oder das medizinische Vorrichtungs-Speicherelement (7) funktionsmäßig mit einem Bewegungssensor verbunden ist.

10. System gemäß einem der vorhergehenden Ansprüche, wobei die Freigabevorrichtung (3) ein Erfassungselement aufweist und konfiguriert ist, wenn mindestens eines von dem Patienten-Speicherelement (1) und dem medizinischen Vorrichtungs-Speicherelement (7) durch das Erfassungselement als in der Umgebung der medizinischen Vorrichtung (2) angeordnet erfasst wird, einen Betriebsmodus zu starten, in dem die Identifizierungsinformationen und die Verwendungsinformationen automatisch von der Freigabevorrichtung (3) entsprechend von dem Patienten-Speicherelement (1) und dem medizinischen Vorrichtungs-Speicherelement (7) gesammelt werden.

11. System gemäß einem der vorhergehenden Ansprüche, wobei die Freigabevorrichtung (3) funktionsmäßig mit einem Betriebsmodul der medizinischen Vorrichtung (2) verbunden ist, wobei die Freigabevorrichtung (3) ferner konfiguriert ist, sofern in dem Schritt des Analysierens gefunden wird, dass die Anwendung der medizinischen Vorrichtung (2) für den Patienten erlaubt ist, das Freigabesignal mit einem Betriebsfreigabesignal zu versehen, das an das Betriebsmodul zum Freigeben der Verwendung einer Betriebsfunktion der medizinischen Vorrichtung (2) übermittelt wird.

12. System gemäß einem der vorhergehenden Ansprüche, wobei die medizinische Vorrichtung (2) eine Vorrichtung oder eine Kombination von Vorrichtungen ist, die aus der folgenden Gruppe ausgewählt ist/sind: eine Stechvorrichtung für die Punktierung einer Patientenhaut, eine Probenentnahmevorrichtung für die Entnahme einer Körperflüssigkeitsprobe, eine Testvorrichtung zum Testen einer Körperflüssigkeit und eine medizinische Injektionsvorrichtung zur parenteralen Verabreichung einer Flüssigkeit.

13. Verfahren zum Bedienen eines medizinischen Systems (2), wobei das Verfahren die Schritte umfasst:
- Bereitstellen eines Patienten-Speicherelements (1), wobei das Patienten-Speicherelement (1) Identifizierungsinformationen aufweist, die einen Patienten identifizieren,
- Bereitstellen eines medizinischen Vorrichtungs-Speicherelements (7) auf einer medizinischen Vorrichtung (2), wobei die medizinische Vorrichtung (2) für eine kontaminierende Patientenanwendung konfiguriert ist,
- Bereitstellen von Verwendungsinformationen auf dem medizinischen Vorrichtungs-Speicherelement (7),
- Bereitstellen einer Freigabevorrichtung (3), und
- in der Freigabevorrichtung (3), Empfangen der Identifizierungsinformationen von dem Patienten-Speicherelement (1) und der Verwendungsinformationen von dem medizinischen Vorrichtungs-Speicherelement (7) und Analysieren der Verwendungsinformationen,
**dadurch gekennzeichnet, dass** das Verfahren in der Freigabevorrichtung (3) ferner die folgenden Schritte umfasst:
- Ausgeben eines Freigabesignals, sofern im Schritt des Analysierens der Verwendungsinformationen gefunden wird, dass die Anwendung der medizinischen Vorrichtung (2) für den Patienten erlaubt ist, und
- Analysieren der Identifizierungsinformationen, sofern im Schritt des Analysierens der Verwendungsinformationen gefunden wird, dass die Anwendung der medizinischen Vorrichtung (2) für den Patienten nicht erlaubt ist, und Ausgeben eine Freigabesignals, sofern in dem Schritt des Analysierens der Identifizierungsinformationen gefunden wird, dass die Anwendung der medizinischen Vorrichtung (2) für den Patienten erlaubt ist.

## Revendications

1. Système médical, comprenant :
- un élément d'enregistrement de patient (1), l'élément d'enregistrement de patient (1) étant affecté un patient en incluant des informations d'identification identifiant le patient,
- un dispositif médical (2), ce dispositif médical (2) étant conçu pour être appliqué sur un patient contagieux,
- un élément d'enregistrement de dispositif médical (7), cet élément d'enregistrement de dispositif médical (7) étant prévu sur le dispositif médical (2) et comprenant des informations d'utilisation, et
- un dispositif d'autorisation (3), le dispositif d'autorisation (3) étant conçu pour
- recevoir les informations d'identification en provenance de l'élément d'enregistrement de patient (1) et les éléments d'utilisation en provenance de l'élément d'enregistrement de dispositif médical (7) et
- analyser les informations d'utilisation,
**caractérisé en ce que** le dispositif d'autorisation (3) est en outre conçu pour
- si, dans l'étape d'analyse des informations d'utilisation, il s'avère que l'application du dispositif médical (2) au patient est permise, émettre un signal d'autorisation et
- si, dans l'étape d'analyse des informations d'utilisation, il s'avère que l'application du dispositif médical (2) au patient n'est pas permise, analyser les informations d'identification et si, dans l'étape d'analyse des informations d'identification, il s'avère que l'application du dispositif médical (2) au patient est permise, émettre un signal d'autorisation.

2. Système selon la revendication 1, dans lequel les informations d'utilisation comprennent au moins un type d'information sélectionné dans le groupe suivant : autres informations d'identification, informations de désinfection et informations de non-utilisation.

3. Système selon la revendication 1 ou 2, dans lequel le dispositif d'autorisation (3) est au moins partiellement mis en oeuvre sous forme d'un dispositif intégré avec un élément entre l'élément d'enregistrement de patient (1) et l'élément d'enregistrement de dispositif médical (7).

4. Système selon l'une quelconque des revendications précédentes, dans lequel au moins un élément entre l'élément d'enregistrement de patient (1) et l'élément d'enregistrement de dispositif médical (7) est un élément d'enregistrement RFID.

5. Système selon l'une quelconque des revendications précédentes, dans lequel au moins un élément entre l'élément d'enregistrement de patient (1) et l'élément d'enregistrement de dispositif médical (7) est un élément d'enregistrement passif.

6. Système selon l'une quelconque des revendications précédentes, dans lequel au moins un élément entre l'élément d'enregistrement de patient (1) et l'élément d'enregistrement de dispositif médical (7) est un élément d'enregistrement actif.

7. Système selon l'une quelconque des revendications précédentes, dans lequel l'élément d'enregistrement de patient (1) est prévu sur un élément d'attachement au patient (4).

8. Système selon l'une quelconque des revendications précédentes, dans lequel l'élément d'enregistrement de dispositif médical (7) est un article jetable appliqué de manière détachable sur le dispositif médical (2).

9. Système selon l'une quelconque des revendications précédentes, dans lequel au moins un élément entre l'élément d'enregistrement de patient (1) et l'élément d'enregistrement de dispositif médical (7) et un élément d'enregistrement (7) est connecté au niveau fonctionnel à un détecteur de mouvement.

10. Système selon l'une quelconque des revendications précédentes, dans lequel le dispositif d'autorisation (3) comprend un élément de détection et est configuré pour démarrer, si au moins un élément entre l'élément d'enregistrement de patient (1) et l'élément d'enregistrement de dispositif médical (7) est détecté par l'élément de détection comme étant localisé à proximité du dispositif médical (2), un mode opératoire dans lequel les informations d'identification et les informations d'utilisation sont collectées automatiquement par le dispositif d'autorisation (3) respectivement dans l'élément d'enregistrement de patient (1) et l'élément d'enregistrement de dispositif médical (7).

11. Système selon l'une quelconque des revendications précédentes, dans lequel le dispositif d'autorisation (3) est connecté au niveau fonctionnel à un module d'actionnement du dispositif médical (2), le dispositif d'autorisation (3) étant en outre conçu pour fournir si, dans l'étape d'analyse, il s'avère que l'application du dispositif médical (2) au patient est permise, le signal d'autorisation avec un signal d'autorisation d'actionnement qui est fourni au module d'actionnement pour autoriser l'utilisation d'une fonction d'actionnement du dispositif médical (2).

12. Système selon l'une quelconque des revendications précédentes, dans lequel le dispositif médical (2) est un dispositif ou une combinaison de dispositifs sélectionné dans le groupe suivant : un dispositif à lance pour piquer la peau d'un patient, un dispositif de prélèvement d'échantillons pour prélever un échantillon de fluide physiologique, un dispositif de test pour tester un fluide physiologique et un dispositif d'injection médicale pour administrer le fluide par voie parentérale.

13. Procédé d'actionnement d'un système médical (2), ce procédé comprenant les étapes de :
- prévision d'un élément d'enregistrement de patient (1), l'élément d'enregistrement de patient (1) comprenant des informations d'identification identifiant un patient,
- prévision d'un élément d'enregistrement de dispositif médical (7) sur un dispositif médical (2), le dispositif médical (2) étant conçu pour application sur un patient contagieux,
- prévision d'informations d'utilisation sur l'élément d'enregistrement de dispositif médical (7),
- prévision d'un dispositif d'autorisation (3) et
- ce procédé comprenant en outre dans le dispositif d'autorisation (3) les étapes suivantes de
- réception des informations d'identification en provenance de l'élément d'enregistrement de patient (1) et des informations d'utilisation en provenance de l'élément d'enregistrement de dispositif médical (7) et
- analyse des informations d'utilisation,
**caractérisé par le fait que**, dans le dispositif d'autorisation (3), le procédé comprend en outre les étapes suivantes :
- si, dans l'étape d'analyse des informations d'utilisation, il s'avère que l'application du dispositif médical (2) au patient est permise, édition d'un signal d'autorisation et
- si, dans l'étape d'analyse des informations d'utilisation, il s'avère que l'application du dispositif médical (2) au patient n'est pas permise, analyse des informations d'utilisation et si, dans l'étape d'analyse des informations d'utilisation, il s'avère que l'application du dispositif médical (2) au patient est permise, édition d'un signal d'autorisation.
